# EUROPEAN PATENT SPECIFICATION

(11) **EP 2 626 111 B1**
(45) Date of publication and mention of the grant of the patent: **17.08.2016**
(21) Application number: 12154506.5
(22) Date of filing: 08.02.2012
(51) Int. Cl.: A61N 1/36, A61N 1/372

(54) **Medical system and method for planning a neuromodulation therapy**
Medizinisches System und Verfahren zur Planung einer Neuromodulationstherapie
Système médical et procédé de programmation d'une thérapie de neuromodulation

(43) Date of publication of application: 14.08.2013
(73) Proprietor: Medtronic Bakken Research Center B.V., 6229 GW Maastricht (NL)
(72) Inventor: Martens, Hubert Cécile François, 5611 ND Eindhoven (NL); Åström, Mattias Bengt Johan, 72341 Västerås (SE)
(74) Representative: Hughes, Andrea Michelle

(56) References cited:
- WO-A1-2009/073891
- US-A1- 2006 017 749
- US-A1- 2011 172 744

## Description

### Description

The description relates to a medical system, in particular medical device, and method for planning and/or tuning a neuromodulation therapy, in particular deep brain stimulation. The invention is set out in the appended claims.

Deep brain stimulation is an established therapy for treatment of movement disorders such as essential tremor, Parkinson's disease, and dystonia, and other neurological disorders. A typical system for applying deep brain stimulation comprises an implantable neurostimulation probe having at least one electrode which is adapted to generate an electrical stimulation field. The stimulation field is applied to selected brain regions in order to directly change brain activity in a controlled manner. The location and distribution of the stimulation field influences the therapeutic effect.

During implantation of the probe, the physician or surgeon recognizes by means of immediate patient feedback to the stimulation, such as reduction or increase of local tremor, whether the location of the probe results in a desired effect or undesired side-effect. As such, known methods for planning the implantation of the neuromodulation probe or adapting electrical stimulation parameters are primarily based upon a trial and error approach which, however, disregards delayed feedback or clinical outcome. Particularly, common methods of planning a neuromodulation therapy do not include considering long-term effects or side-effects like impaired speech (dysarthria) or introduction of hypomania which may develop over time. Thus, programming of a deep brain stimulation system is performed without any direct feedback which allows for an assessment as to whether the adapted stimulation field produces long-term beneficial effects and/or avoids long-term side-effects. The same applies for tuning a neuromodulation therapy, i.e. for developing an optimal stimulation field distribution for a specific patient.

WO 2009/073891, US 2011/0172744 and US 2006/0017749 describe systems and methods for providing targeted neural stimulation therapy. In particular US 2006/0017749 discloses computing a volume of influence region for deep brain stimulation.

It is an object of the present description to provide for a medical system and a method for planning and/or tuning a neuromodulation therapy which aids in adapting a location and/or distribution of a neuromodulation probe's stimulation field such that short-term as well as long-term side effects are reduced or avoided.

This object is solved according the present invention by a system as claimed in claim 1 and a method as claimed in claim 9.

In particular, this object is solved by a medical system, in particular medical device, for planning and/or tuning a neuromodulation therapy, the system comprising:
- a database means for storing a plurality of data sets, each data set comprising:
   - meta data, e.g. patient data, in particular relating to gender data, age data, local field recordings data, microelectrode recordings data, tissue fiber structures data, demographics data and/or anatomy data of a patient, and
   - spatial data relating to the distribution of a stimulation field of a neuromodulation probe;
- input means for entering at least one filter criterion;
- filter means for applying the at least one filter criterion on the plurality of data sets to generate a filtered subset of the data sets;
- merging means for merging the spatial data of the filtered subset to generate a functional data set;
- visualization means for computing a three dimensional model of the functional data set and for producing a visual an image of the three dimensional model.

The invention is based upon the idea to use a database means for storing statistical data to determine stimulation regions which are expected to show a certain clinical outcome (positive or negative). The database means may contain statistical data of patients which have been treated with a neuromodulation probe, in particular a neuromodulation probe of a deep brain stimulation system. This data may be used to generate dynamic functional brain atlas (dFBA). E.g. the dFBA uses a certain amount of data sets, each data set representing a specific patient of a group of patients which have been treated with the neuromodulation probe. Each data set may comprise meta data of a specfic patient and/or patient group and spatial data about the distribution of a stimulation field which has been generated during therapy of said patient and/or patient group. The meta data may also include information about the clinical outcome of each patient.

Each data set may also include spatial data related to the stimulation field applied to the specific patient which provided the meta data of said data set. The distribution of the stimulation field may be determined by setting up a coordinate system, preferably a Cartesian coordinate system, aligned to the probe and defining a probe grid. Each point of the probe grid which includes spatial data related to the stimulation field defines the volume of the stimulation field. Spatial data may be assigned only to probe grid points which are covered by the stimulation field. Hence, only spatial data which has been assigned to probe grid points, i.e. which represent probe grid points covered by the stimulation field, may be combined with meta data in a data set.

By filtering the data sets, a filtered subset of the data sets is generated which includes data sets of patients matching the entered filter criterion or criteria. Thus, the invention allows for a user, e.g. a physician, in particular a neurologist, to identify specific criteria for including or excluding data sets that may or may not be of interest.

The merging means may allow for computing average scores related to clinical outcome on the basis of a filtered subset of data sets. The filtered subset of data sets may include data sets of different patients matching the selected filter criterion or criteria. For example, a filter criterion may be applied to the data sets, in particular the meta data of the data sets, which generate a filtered subset of the data sets wherein the filter criterion relates to the age of a patient and the clinical outcome of that patient. Each data set also contains spatial data of the stimulation field applied to the respective patient. The meta data of a specific data set is assigned to all spatial data values. In other words each point of the probe grid or a main grid, as explained thereinafter, may be assigned the same meta data. By merging a specific data value, e.g. a data value relating to the clinical outcome, of the data sets included in the filtered subset, the system can provide an assessment based on the statistical data of the dFBA as to whether stimulation of a specific region has beneficial effects or undesired side-effects.

The present description advantageously allows for guidance during surgical planning of a neuromodulation therapy, in particular of probe implantation, intra-operative test stimulation, and postoperative stimulation tuning.

Surgical planning may be aided by at least one three dimensional model/object representing a functional map related to best clinical outcome together with at least one functional map displaying regions that are likely to produce stimulation-induced side-effects. These functional maps may be used to identify anatomical target areas where stimulation will reduce the symptoms of the disease at the same time as side-effects are avoided. Thus, the access point for implanting the neuromodulation probe, the angle of the neuromodulation probe in relation to a body axis or body plane, in particular to the AC-PC (anterior commissure-posterior commissure) plane, and/or the stimulation parameters, e.g. stimulation current, stimulation amplitude and/or stimulation frequency, may be determined by means of the visualization of the target area, preferably the functional map (three dimensional model). The stimulation parameters may be controlled per each electrode of an electrode array attached to the probe, thereby adjusting the distribution of the stimulation field.

In addition, the three dimensional model/object which is computed on the basis of the functional data and which represents a selected functional map may provide a visual representation of data of local field recordings or other recorded data. The functional map may be displayed together with at least one image of patient-specific anatomy. Displaying local field recordings or other recorded data may also be useful for identifying optimal stimulation areas or, respectively, stimulation targets. Functional maps will further aid a user, e.g. a physician, in particular a neurologist, during intra-operative test stimulation by providing information regarding the location of the neuromodulation probe. The location of the probe may be derived from immediate feedback of stimulation-induced effects and side-effects together with the functional maps relating to stimulation-induced effects and side-effects observed in a filtered subset of data sets, i.e. relating to stimulation-induced effects and side-effects observed in a selected comparison group of patients.

Moreover, the location of the neuromodulation probe may possibly be derived from recorded local field potentials that are compared with at least one three dimensional model/object, i.e. at least one functional map, displaying previously recorded local field potentials.

The postoperative stimulation tuning may also be aided by functional maps in order to tune the stimulation field towards areas that are likely to produce beneficial effects without side-effects. The dFBA contains information regarding delayed effects and side-effects that may not be immediately tested during tuning. Thus, functional maps may therefore be used to avoid not only immediate, but also delayed, undesired clinical effects, or to alleviate, preferably to avoid, symptoms on which neurostimulation has a delayed effect.

The functional maps or, respectively, the functional data set which forms the basis of the three dimensional model/object representing the functional map, may be generated dynamically. In other words, based on the entered input data, a specific subset of data sets from the database, herein referred to as dFBA, may be filtered, merged and visualized as an image of the three dimensional model/object. Because different filter criteria may be entered, the inventive system provides for a dynamic generation and visualization of the functional data, i.e. functional maps, which represent the functional data merged from the filtered subset of data sets. Dynamically created functional maps of e.g. stimulation fields related to "Best clinical outcome" together with maps of e.g. local field potentials may be an important tool not only for tuning but also for understanding the underlying mechanisms of neuromodulation therapy, in particular deep brain stimulation, which is essential for a more widespread use of said kind of therapy in different neurological disorders.

It is preferred that the system comprises registration means for mapping the three dimensional model of the functional data set on at least one patient-specific medical image, in particular a magnetic resonance image, whereby the visualization means is adapted to produce a visual image of the three dimensional model together with the patient-specific medical image. In other words, the functional data set generated from the filtered subset of data can be displayed in relation to a patient-specific medical image, thereby, aiding in determining a location and/or distribution of the stimulation field which has been developed as having a beneficial therapeutic effect and avoiding undesired side-effects. The three dimensional model of the functional data substantially represents a specific functional map wherein the geometry of the functional map depends on the filter criterion applied to the plurality of data sets, i.e. the dFBA. The physician may enter the filter criterion and, thus, decide upon the geometry of the three dimensional model, i.e. the functional map, to be visualized or displayed. The three dimensional model may be displayed in 2D or 3D together with the patient-specific medical image.

In a preferred embodiment, the spatial data comprises a plurality of data values, each data value being assigned to a specific location in a main grid, preferably a blank Cartesian main grid. Moreover, the meta data may be mapped to the main grid. Additionally, the volume of the stimulation field may be transformed and/or interpolated from the probe grid points to the main grid points.

The main grid may represent a graphical visualization of the database, herein referred to as dFBA, containing the plurality of data sets. Each point of the main grid is represented in the database by a spatial data value. Preferably, the main grid is directly mapped onto patient-specific medical images, e.g. magnetic resonance images (MRI) or computer tomographic images, instead of an anatomical atlas. Thus, the user is allowed to identify the anatomical structures.

Further, the merging means may be adapted to calculate an average score of each data value of the filtered subset which is assigned to the same location in the main grid. The data value may comprise information about the clinical outcome of a neuromodulation therapy. Thereby, a three dimensional model (functional map) can be generated and visualized which shows stimulation regions which have been developed as having beneficial effects for a certain group of patients relating to the filtered subset of data sets.

The physician may select a certain group of patient data contained in the database which match the entered filter criterion. The average of the data values of the filtered subset of data sets related to clinical outcome at a specific stimulation location, in particular a specific point of the main grid, may then be calculated by the merging means. The points of the main grid which have an averaged data value above a certain threshold may be visualized to display the three dimensional model. Thus, for example a three dimensional model or a functional map may be visualized together with a patient-specific medical image which corresponds to averaged data values representing the best clinical outcome.

The data values of the filtered subset of data sets which are mapped to a specific location in the main grid and of which an averaged data value may be calculated, may comprise a score which is assigned to the spatial data after clinical assessment. The score may consider effects of a neuromodulation therapy according to keywords such as "best", "good", "moderate", "bad" and "worst". Side-effects may be scored by keywords like "intolerable", "severe", "some" and "minor". The data value relating to clinical outcome may be represented by a number ranging from "0" to "4", each number being assigned to a first keyword relating to effects and a second keyword relating to side-effects.

Additionally, the spatial data may be represented by a matrix, in particular a three dimensional matrix. In order to allow for a high resolution, it is preferred that for each point in the main grid the distance to its immediate neighbour points does not exceed 1 mm, particularly 0.75 mm, particularly 0.5 mm, particularly 0.25 mm, particularly 0.1 mm.

The filter means may be adapted to receive at least one Boolean expression related to the meta data. Additionally, the filtered subset of data sets may be generated at least partially based on the Boolean expression. Thus, the functional map represented by the three dimensional model is easily selectable by the physician who enters the respective filter criterion.

In a further preferred embodiment, the system comprises a simulation means for simulating a stimulation field of the neuromodulation probe, wherein the visualization means is adapted to visualize the stimulation field in relation to the three dimensional model of the functional data set. The visualization of the stimulation field aids the physician in determining the stimulation parameters which control the distribution of the stimulation field. The simulation parameters may be controlled such that the three dimensional model/object, e.g. representing a functional map for best clinical outcome of a specific group of patients, is optionally covered by the stimulation field.

A further aspect of the present description concerns a method for planning and/or tuning a neuromodulation therapy, the method comprising:
- storing a plurality of data sets, each data set comprising
   - meta data, e.g. patient data, in particular relating to gender data, age data, local field recordings data, microelectrode recordings data, tissue fibre structures data, demographics data and/or anatomy data of a patient, and
   - spatial data relating to the distribution of a stimulation field of a neuromodulation probe;
- entering at least one filter criterion;
- applying the at least one filter criterion on the plurality of data sets to generate a filtered subset of the data sets;
- merging the spatial data of the filtered subset to generate a functional data set;
- computing a three dimensional model of the functional data set; and
- producing a visual an image of the three dimensional model.

The system or device, according to the present description may comprise a workstation on which at least one of the database means, the input means, the filter means, the merging means, the visualization means, the registration means and the simulation means are installed. The system/device may further comprise a screen, in particular a flat screen and/or touch screen, for displaying the image of the three dimensional model, preferably together with the patient-specific medical image.

The system/device may be embodied as a computer program product.

The system and/or method according to the present description are advantageously adapted for programming a deep brain stimulation system, in particular a deep brain stimulation system as described in detail hereinafter.

The present invention is set out in the appended claims that follow. The embodiments, aspects and examples of the description that do not fall within the scope of said claims are provided for illustrative purposes only and do not form part of the invention.

Further details and advantages of the present description shall be described hereinafter with respect to the drawings:
- FIG. 1:: a schematical drawing of a neurostimulation system for deep brain stimulation (DBS);
- FIG. 2:: a further schematical drawing of a probe neurostimulation system for deep brain stimulation (DBS) and its components;
- FIG. 3:: a schematical drawing of a probe system according to the present description;
- FIG. 4:: a schematical drawing of an image guided support system;
- FIG. 5:: an illustration of a main grid registered to a patient-specific medical image;
- FIG. 6:: an illustration of a neuromodulation probe and a simulated stimulation field within a probe grid;
- FIG. 7:: the illustration of the simulated stimulation field of FIG. 6 registered within the main grid of FIG. 5;
- FIG. 8:: a visualization of a functional map, in particular a target stimulation field, within a patient specific medical image;
- FIG. 9:: the visualization as shown in FIG. 8 including three dimensional anatomy structures;
- FIG. 10:: the visualization as shown in FIG. 9 including a neuromodulation probe;
- FIG. 11:: the visualization as shown in FIG. 10 including a simulated stimulation field.

Deep brain stimulation (DBS) is an established therapy for treatment of movement disorders such as essential tremor, Parkinson's disease (PD), and dystonia, and is being used on an experimental level for a range of other neurological disorders. However, on a detailed level there is no consensus regarding optimal stimulation targets for any of these disorders. It is not clear if one optimal target exists for each of these neurological disorders or if there are multiple targets areas that regulate various symptoms of each disease. Thus, planning and programming of DBS is complicated and to some degree experimental also for treatment of diseases that are approved by the Food and Drug Administration. In order to improve and facilitate the planning, intra-operative stimulation/recordings, and postoperative programming of DBS, a medical system/device for planning and/or running a neuromodulation therapy, e.g. image-guided support systems 10, may serve as a valuable tool.

Such an image guided support system 10 could be included into a neuromodulation/neurostimulation system 100. As an alternative a respective system could be provided as a separate unit.

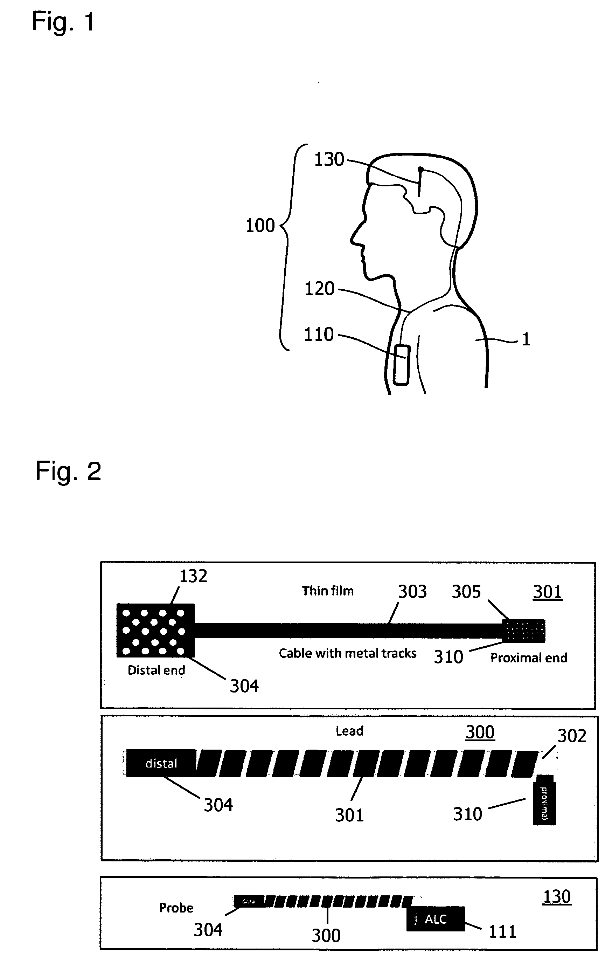

One embodiment of a neurostimulation system 100 for deep brain stimulation (DBS) is shown in FIG. 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

FIG. 2 further illustrates a typical architecture for a Deep Brain Stimulation probe 130 that comprises a DBS lead 300 and an Advanced Lead Connector (ALC) element 111 comprising electronic means to address electrodes 132 on the distal end 304 of the DBS lead 300. The DBS lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 111. The ALC element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrode 132 to a designated proximal contact 305.

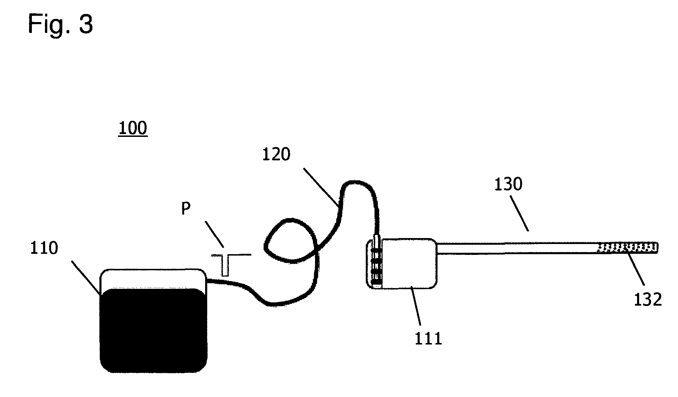

FIG. 3 shows schematically and in greater detail an embodiment of a system 100 for brain applications, here for neurostimulation and/or neurorecording as a deep brain stimulation system 100 as shown in FIGS. 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 111. The controller 110 can be an implantable pulse generator (IPG) 110.

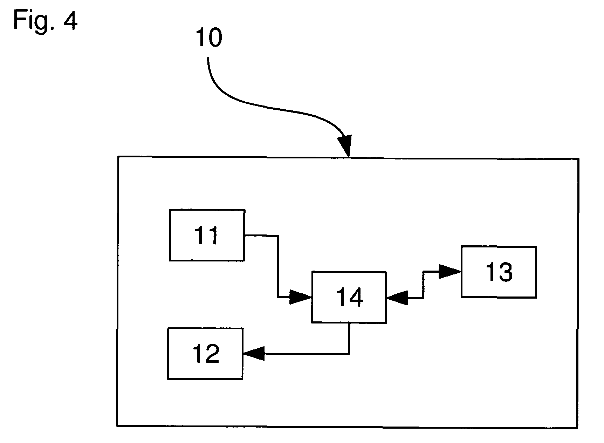

FIG. 4 shows a respective embodiment of the image guided support system 10. This image guided support system 10 comprises several components, namely a keyboard 11, a display 12, a memory 13 and a central processing unit (CPU) 14. The image guided support system 10 is designed such that it can receive a user input by means of the keyboard 11 and pass respective input data on to the central processing unit 14. The central processing unit 14 can use programs (software) stored in the memory 13 to process the input data and to generate an output which can be displayed on the display 12. In one embodiment of the present invention the memory 13 may contain programs as well as data to be processed, e.g. data sets k, k', k". Alternatively, several memories 13 might be used to store different type of data, e.g. one memory 13 for the programs and one memory 13 for the data to be processed.

In the embodiment of FIG. 4, the memory 13 holds a plurality of data sets k, k', k" wherein each data set comprises meta data v_{D}, v_{D'}, v_{D"} and spatial data in the form of a spatial data matrix D, D', D". There are numerous ways of modelling respective data sets k, k', k". The following description should be considered one exemplary approach to store the data contained in the data sets k, k', k".

The data set k could be modelled as a vector with the elements {v_{D}, D}. Accordingly, k' could be a vector with the element {v_{D'}, D'} and k" with the elements {v_{D"}, D"}.

Thus, each data set k, k', k" comprises specific meta data, e.g. the first meta data vector v_{D}, the second meta data vector v_{D'} and the third meta data vector v_{D"}, and spatial data, e.g. the first data matrix D, the second data matrix D' and the third data matrix D". The meta data vectors v_{D}, v_{D'}, v_{D"} might contain information related to the patient's anatomy, tissue fibre structures, patient demographics, local field potential recordings (LFP), microelectrode recordings (MER), (side-)effects, treated neurological disorders, symptoms, treatment parameters and/or clinical evaluations.

The exemplary first meta data vector v_{D} might contain the values {male, 60, positive, PD} which might indicate that a male patient aged 60 having Parkinson's disease has been treated with deep brain simulation whereby the overall clinical outcome is considered positive. Similarly, the second meta data vector v_{D'} might contain the values {female, 55, positive, PD} to indicate that a female patient aged 55 having Parkinson's disease has been treated with a positive clinical outcome. The third meta data vector v_{D"} might have the values {female, 50, negative, PD} to indicate that a female patient aged 50 having Parkinson's disease has been treated with a negative clinical outcome.

The spatial data might be modelled in spatial data matrix D having a plurality of data values d_{x,y,z}. It is obvious that respective data values d_{x,y,z}, can be mapped on a Cartesian coordinate system. FIG. 4 shows a main grid 30 having an X-axis 31, Y-axis 32 and Z-axis 33. An origin 35 of the main grid 30 holds the coordinates {0, 0, 0}. Accordingly, the data value d_{0,0,0} would be mapped to the origin 35 of the main grid 30. For the person skilled in the art it should be obvious where other data values d_{x,y,z} of the spatial data matrix D will be mapped to in the main grid 30.

In one embodiment, the spatial data matrix D will hold data values d_{x,y,z} = 0 or d_{x,y,z} = 1, wherein the value 1 indicates that within a particular deep brain stimulation session, a particular region has been stimulated and wherein 0 indicates that the respective region within the main grid 30 has not been stimulated. Thus, the spatial data matrix D might encode a three dimensional model/object of a respective stimulation field. Similarly, the other spatial data matrices D', D" might encode a three dimensional model of a different stimulation field used with e.g. different patients.

In one embodiment, each data set k, k', k" might represent one treatment of a particular patient, wherein the stimulated region of the brain and numerous information about the patient, the outcome of the treatment and treatment itself are recorded. It is an objective of the present invention to visualize respective results such that they can be used for planning and/or tuning a neuromodulation therapy. In other words, the data sets k, k', k" provide data such that the dFBA can generate different functional maps which can be used for planning and/or tuning a neuromodulation therapy.

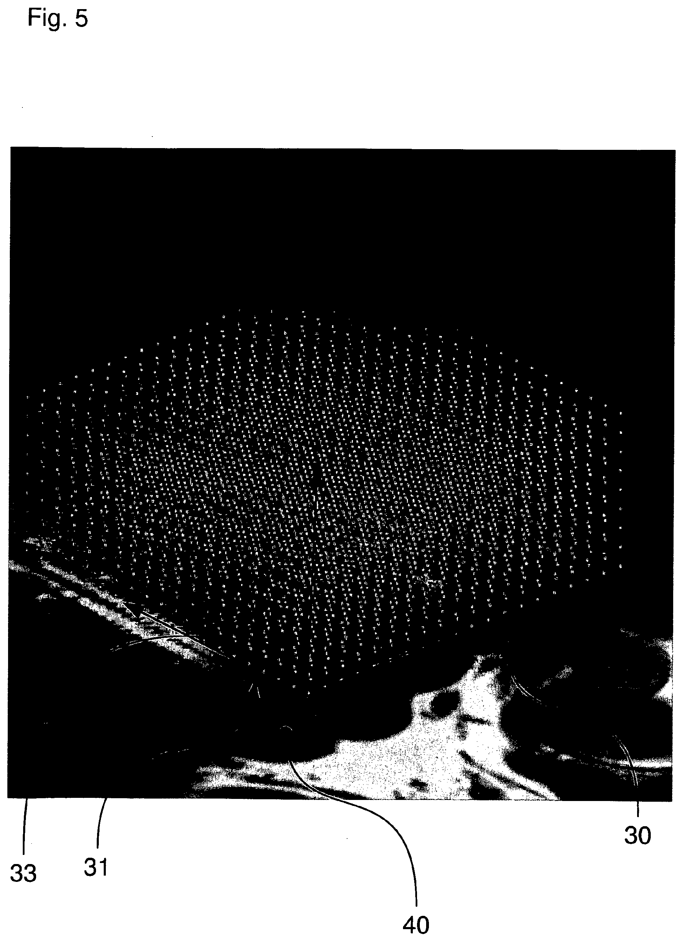

Preferably, the functional maps of the dFBA are shown together with images representing the anatomy of a patient to be treated. For high quality results, the main grid 30 should be registered (e.g. scaled and aligned) with the patient's specific medical images, as shown in Fig. 5.

Concerning this, several approaches are available. A first approach uses a template medical image data set. The main grid 30 is first (before any functional data is mapped into the main grid) registered with the template images by aligning the main grid with the AC-PC plane, and placing the origin 35 at the midcommissural point (MCP) i.e. at a point in the middle between the AC and PC. Thus, the AC and PC must be identified in the template images, together with the mid-sagittal plane. After this one time procedure, the main grid 30 is registered to patient-specific anatomy simply by registering the patient-specific images with the template image data set. The derived transformation matrix is then applied to the main grid 30. In order to get an accurate and precise registration, elastic registration can be used where the algorithms are adjusted in order to optimize the registration of the target structures where the DBS electrodes are located.

A second registration approach may immediately align the main grid 30 with the anatomy of the patient. In this approach the main grid 30 should be aligned with the patient's AC-PC plane. Thus, the AC and PC must be identified in the patient-specific images, together with the mid-sagittal plane.

Anatomical landmarks might be used to improve either one of the approaches. One landmark for defining the subthalamic nuclues (STN) is the posterior point of the STN at the level of the centre of the red nucleus. Another landmark that could be used as origin is the centre of the STN at the level of the centre of the red nucleus. Finding the landmarks and marking them may be performed manually by the user. After the landmarks have been selected they can be mapped to the corresponding landmarks registered in the main grid 30.

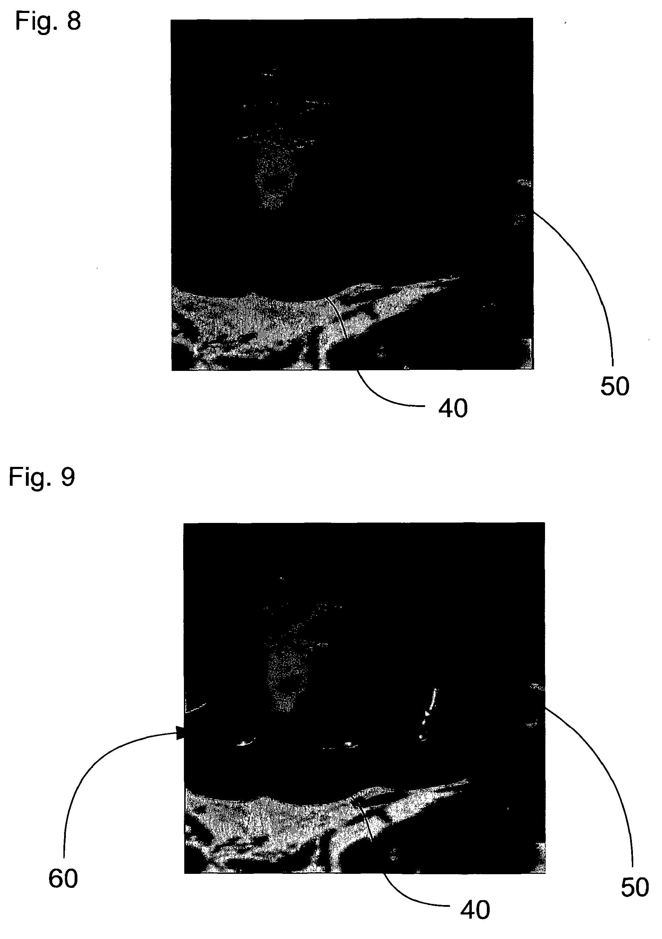

Once the main grid 30 has been registered, it is possible to visualize the information contained in the data sets k, k', k" as shown in the FIGS. 8 to 11. This could be done by simply mapping the spatial data of one data set k, k', k" on the main grid 30. Preferably, the image guided support system implements a dFBA by applying a filter to the plurality of data sets k, k', k" stored in the memory 13. Thereby, a subset of the data sets k, k', k" is selected which can then be visualized.

The filter criterion or criteria can be expressed by Boolean operators. For example, only those data sets k, k', k" where Parkinson's disease was treated and the clinical outcome was considered positive might be relevant. The respective Boolean expression could read as follows: "disease='PD' AND clinical outcome='positive'". With the meta data vectors v_{D}={male, 60, positive, PD}, v_{D'}={female, 55, positive, PD} and v_{D"} {female, 50, negative, PD}, only the first data set k and the second data set k' match the respective filter, i.e. the abovementioned Boolean expression. Thus, only these data sets k, k' will be used to generate a functional map. E.g. the corresponding first and second spatial data matrix D, D' will be added whereby only those points within the main grid 30 will be considered worth treating where the resulting value equals 2. With this result it is very simple to generate a three dimensional object/model of a preferred treatment volume and to visualize the respective three dimension model as shown in FIGS. 8 and 9 (target stimulation field 50). Additional information might be displayed as shown, e.g. in FIG. 9, wherein a three dimensional anatomy 60 is included.

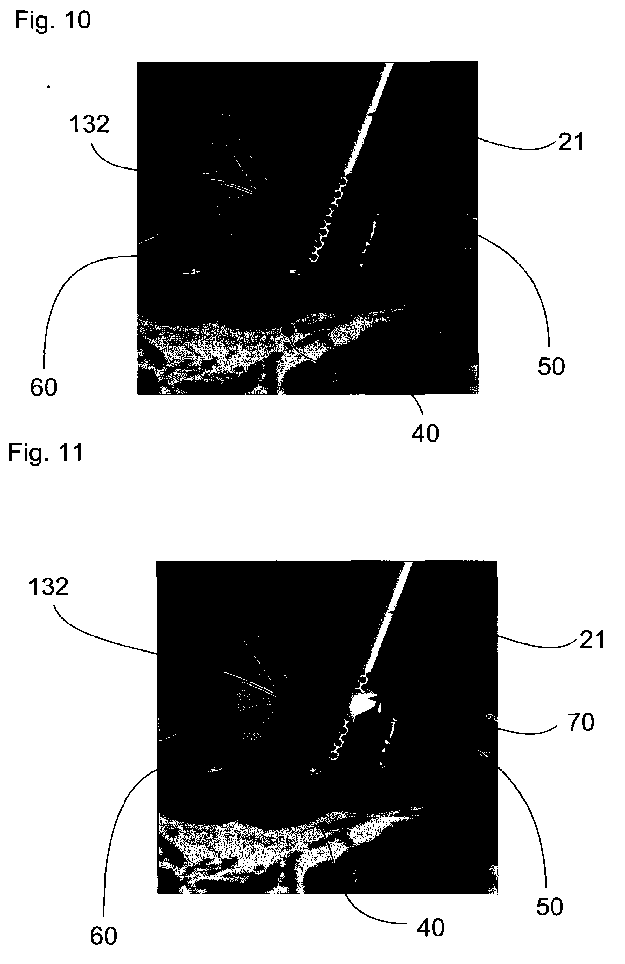

Preferably, the image guided support system 10 also provides a simulation of a probe 21 for DBS (FIG. 11) and a simulated stimulation field 70 (FIG. 2).

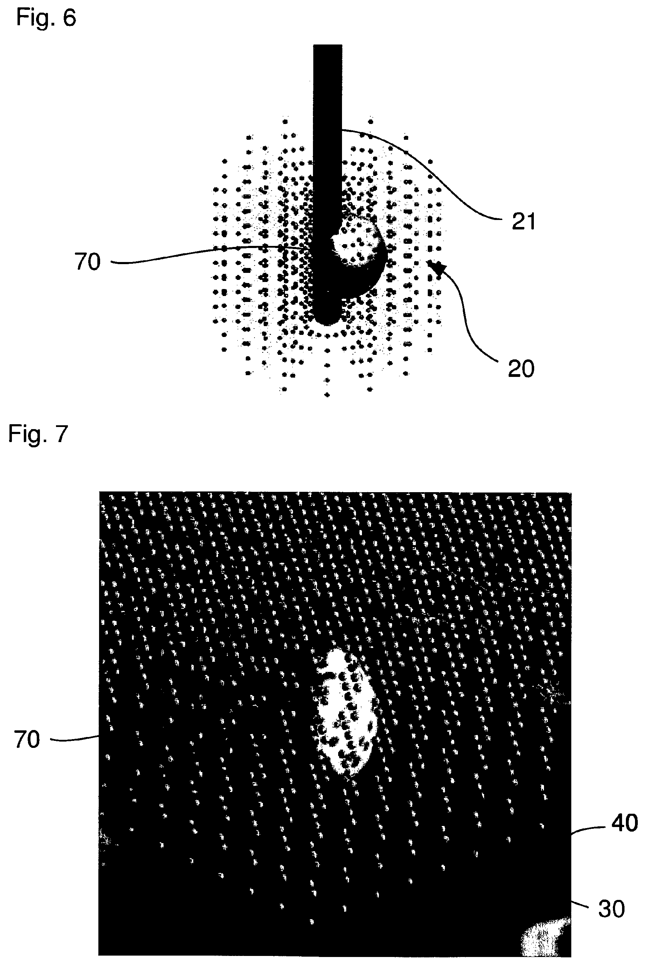

For doing so, a simulated stimulation field 70 of the probe 21 might be generated within a probe grid 20, e.g. FIG. 6, wherein the probe grid 20 is then registered within the main grid 30 to generate an appropriate model of the simulated field 70 as shown e.g. in FIG. 7. Depending on the setting, the probe 21 might also be visualized as shown in FIGs. 10 and 11.

In another embodiment the functional map could include e.g. patients older than 60 years old, who had DBS surgery performed more than 3 years ago, suffering from 'intolerable', 'severe' or 'minor' dysarthria. The image guided support system 10 would then display the preferred target stimulation field 50 for this patient group.

Another example would be to show a functional stimulation map of all patient with best overall clinical outcome (e.g. UPDRS: Unified Parkinson Disease Rating Scale) stimulated with an amplitude below e.g. 0.3 mA. A third example would be to visualize a functional map of recorded frequencies during local field potential (LFP) recordings, together with maps that display the best overall clinical outcome. The combination of such functional data into one common map may provide information on how to use LFP for finding the optimal location for stimulation in a specific neurological disorder. In addition, functional maps displaying delayed effects of DBS may be visualized in order to avoid stimulation of certain areas in the brain that are likely to produce such effects, or the inverse, to stimulate areas that are likely to induce delayed beneficial effects.

If a user of the image guided support system 10 is interested in looking at a functional map regarding stimulation-induced diplopia (double vision), then the image guided support system 10 will calculate the scores for diplopia at each grid point of the main grid 30, and then visualize an isosurface that includes the grid points that have a diplopia score. The threshold for the isosurface i.e. the severity of diploplia may be adjusted by the user in order to display a map of various severities e.g. only the 'Severe' and 'Intolerable' cases. If a user is only interested in looking at a subgroup of these patients such as only patients with an age above 75, who suffer from hypertension, patients will be excluded who do not meet the Boolean expression during the calculation of the data at the grid points of the main grid 30.

Generally, the steps performed by the image guided support system might be summarized as follows:
1) Registering the main grid 30 onto patient specific anatomy;
2) Receiving Boolean expressions from the user interface to select/filter the data that is included in the main grid 30 in various ways by means of the functional spatial maps, i.e. maps that visualize the data together with patient-specific anatomy;
3) Executing mathematical and/or statistical calculations at each point of the main grid 30, e.g. summing the severity of a certain side-effect and then calculating the severity average of this side effect;
4) Visualizing node points within the main grid 30, wherein the node points have a side-effect average severity above a certain threshold;
5) Providing a 3D isosurface over the selected node points.

In the above described embodiment, the database containing the plurality of data sets k, k', k" is stored in the memory 13 of the image guided support system 10. However, in an alternative embodiment, the database may be hosted on any external device. Thus, the database storing the plurality of data sets k, k', k" does not necessarily have to be part of the device. The database may be in any case part of the whole system, irrespective whether it is embodied in a single device or an arrangement of two or more devices. For example, the system may comprise a computing device, which includes the database means, the input means, the filter means and/or the merging means and which creates the functional maps, i.e. the three dimensional model, and a separated visualization device including the visualization means and being adapted to receive and display the functional maps created in the computing device.

### REFERENCE NUMBERS

- 1: patient
- 10: image guided support system
- 11: keyboard
- 12: display
- 13: memory
- 14: central processing unit (CPU)
- 20: probe grid
- 21: probe
- 30: main grid
- 31: X-axis of the main grid
- 32: Y-axis of the main grid
- 33: Z-axis of the main grid
- 40: patient-specific medical image
- 50: target stimulation field
- 60: three dimensional anatomy
- 70: simulated stimulation field
- 100: neuro stimulation system
- 110: controller
- 111: advanced lead connector element (ALC)
- 120: extension wire
- 130: lead arrangement
- 132: electrodes
- 300: DBS lead
- 301: thin film
- 302: carrier
- 303: cable
- 304: distal end
- 305: interconnect contact
- 310: proximal end
- P: pulse
- D, D', D": spatial data matrix
- d_{x,y,z}: data value of matrix D
- d'_{x,y,z}: data value of matrix D'
- d"_{x,y,z}: data value of matrix D"
- v_{D}, v_{D'}, v_{D"}: meta data vector
- k, k', k": data set

The invention is set out in the following claims.

## Claims

1. A medical system for planning and/or tuning a deep brain neuromodulation therapy, the system comprising:
a database for storing a plurality of data sets, each data set comprising
meta data relating to gender data, age data, local field recordings data, microelectrode recordings data, tissue fibre structures data, demographics data and/or anatomy data of a patient, and
spatial data of distribution of a stimulation field (70) of a neuromodulation probe (21);
input means for entering at least one filter criterion;
filter means configured to apply the at least one filter criterion on the plurality of data sets to generate a filtered subset of the data sets;
merging means configured to merge the spatial data of the data sets of the filtered subset to generate a functional data set; and
visualisation means arranged to compute a three dimensional model (50) of the functional data set and to produce a visual image of the three dimensional model (50).

2. The medical system according to claim 1, wherein the spatial data comprises a plurality of data values, each data value being assigned to a specific location in a main grid (30).

3. The medical system according to claim 2, wherein the merging means are adapted to calculate an average score of each data value of the filtered subset which is assigned to the same location in the main grid (30).

4. The medical system according to any of the preceding claims, further comprising registration means for mapping the three dimensional model (50) of the functional data set on at least one patient-specific medical image, in particular a magnetic resonance image, whereby the visualisation means is adapted to produce a visual image of the three dimensional model (50) together with the patient-specific medical image (40).

5. The medical system according to any of the preceding claims, wherein the spatial data is represented by a matrix, in particular a three dimensional matrix.

6. The medical system according to any of claims 2 to 5, wherein for each point in the main grid the distance to its immediate neighbour points does not exceed 1 mm, particularly 0.75 mm, particularly 0.5 mm, particularly 0.25 mm, particularly 0.1 mm.

7. The medical system according to any of the preceding claims, wherein the filter means is adapted to receive at least one Boolean expression related to the meta data, whereby the filtered subset of data sets is generated at least partially based on the Boolean expression.

8. The medical system according to any of the preceding claims, further comprising a simulation means for simulating a stimulation field (70) of the neuromodulation probe (21), wherein the visualisation means is adapted to produce a visual image of the stimulation field (70) in relation to the three dimensional model (50) of the functional data set.

9. A method for planning a deep brain neuromodulation therapy, the method comprising:
storing a plurality of data sets, each data set comprising
meta data relating to gender data, age data, local field recordings data, microelectrode recordings data, tissue fibre structures data, demographics data and/or anatomy data of a patient; and
spatial data relating to the distribution of a stimulation field (70) of a neuromodulation probe (21);
entering at least one filter criterion;
applying the at least one filter criterion on the plurality of data sets to generate a filtered subset of the data sets;
merging the spatial data of the data sets of the filtered subset to generate a functional data set;
computing a three dimensional model (50) of the functional data set; and
producing a visual image of the three dimensional model (50).

## Patentansprüche

1. Medizinisches System zum Planen und/oder Abstimmen einer tiefen Gehirnneuromodulationstherapie, wobei das System Folgendes enthält:
eine Datenbank zum Speichern mehrerer Datengruppen, wobei jede Datengruppe Folgendes enthält:
Metadaten bezogen auf Geschlechtsdaten, Altersdaten, Daten aus lokalen Feldaufnahmen, Daten aus Mikroelektrodenaufnahmen, Gewebefaserstrukturdaten, demographische Daten und/oder Anatomiedaten eines Patienten, und
räumliche Daten der Verteilung eines Reizfeldes (70) einer Neuromodulationssonde (21);
Eingabemittel zum Eingeben mindestens eines Filterkriteriums;
Filtermittel, die konfiguriert sind, das mindestens eine Filterkriterium auf die mehreren Datengruppen anzuwenden, um eine gefilterte Teilmenge der Datengruppen zu erzeugen;
Mischmittel, die konfiguriert sind, die räumlichen Daten der Datengruppen der gefilterten Teilmenge zu mischen, um eine funktionale Datengruppe zu erzeugen; und
Visualisierungsmittel, die ausgelegt sind, ein dreidimensionales Modell (50) der funktionalen Datengruppe zu berechnen und ein visuelles Bild des dreidimensionalen Modells (50) zu erzeugen.

2. Medizinisches System nach Anspruch 1, wobei die räumlichen Daten mehrere Datenwerte enthalten, wobei jeder Datenwert einer spezifischen Position in einem Hauptraster (30) zugeordnet ist.

3. Medizinisches System nach Anspruch 2, wobei die Mischmittel ausgelegt sind, eine durchschnittliche Auswertung für jeden Datenwert der gefilterten Teilmenge zu berechnen, die derselben Position in dem Hauptraster (30) zugeordnet wird.

4. Medizinisches System nach einem der vorhergehenden Ansprüche, das ferner Registrierungsmittel zum Abbilden des dreidimensionalen Modells (50) der funktionalen Datengruppe auf mindestens ein patientenspezifisches, medizinisches Bild, insbesondere auf ein Magnetresonanzbild, enthält, wobei das Visualisierungsmittel ausgelegt ist, ein visuelles Bild des dreidimensionalen Modells (50) zusammen mit dem patientenspezifischen, medizinischen Bild (40) zu erzeugen.

5. Medizinisches System nach einem der vorhergehenden Ansprüche, wobei die räumlichen Daten durch eine Matrix, insbesondere durch eine dreidimensionale Matrix, dargestellt werden.

6. Medizinisches System nach einem der Ansprüche 2 bis 5, wobei für jeden Punkt im Hauptraster der Abstand zu seinem unmittelbaren Nachbarpunkt 1 mm, insbesondere 0,75 mm, insbesondere 0,5 mm, insbesondere 0,25 mm, insbesondere 0,1 mm, nicht überschreitet.

7. Medizinisches System nach einem der vorhergehenden Ansprüche, wobei das Filtermittel ausgelegt ist, mindestens einen auf die Metadaten bezogenen Boole'schen Ausdruck zu empfangen, wobei die gefilterte Teilmenge der Datengruppen mindestens teilweise auf der Basis des Boole'schen Ausdrucks erzeugt wird.

8. Medizinisches System nach einem der vorhergehenden Ansprüche, das ferner ein Simulationsmittel zum Simulieren eines Reizfeldes (70) der Neuromodulationssonde (21) enthält, wobei das Visualisierungsmittel ausgelegt ist, ein visuelles Bild des Reizfeldes (70) in Beziehung zu dem dreidimensionalen Modell (50) der funktionalen Datengruppe zu erzeugen.

9. Verfahren zum Planen einer tiefen Gehirnneuromodulationstherapie, wobei das Verfahren Folgendes umfasst:
Speichern mehrerer Datengruppen, wobei jede Datengruppe Folgendes umfasst:
Metadaten bezogen auf Geschlechtsdaten, Altersdaten, Daten aus lokalen Feldaufnahmen, Daten aus Mikroelektrodenaufnahmen, Gewebefaserstrukturdaten, demographische Daten und/oder Anatomiedaten eines Patienten; und
räumliche Daten bezogen auf die Verteilung eines Reizfeldes (70) einer Neuromodulationssonde (21);
Eingeben mindestens eines Filterkriteriums;
Anwenden des mindestens einen Filterkriteriums auf die mehreren Datengruppen, um eine gefilterte Teilmenge der Datengruppen zu erzeugen;
Mischen der räumlichen Daten der Datengruppen der gefilterten Teilmenge, um eine funktionale Datengruppe zu erzeugen;
Berechnen eines dreidimensionalen Modells (50) der funktionalen Datengruppe; und
Erzeugen eines visuellen Bildes des dreidimensionalen Modells (50).

## Revendications

1. Système médical destiné à planifier et/ou mettre au point une thérapie de neuromodulation cérébrale profonde, le système comportant :
une base de données pour mémoriser une pluralité d'ensembles de données, chaque ensemble de données comportant des métadonnées concernant des données sur le sexe,
des données sur l'âge, des données sur des enregistrements de champs locaux, des données sur des enregistrements de microélectrodes, des données sur des structures de fibres tissulaires, des données démographiques et/ou des données anatomiques d'un patient, et
des données spatiales de répartition d'un champ de stimulation (70) d'une sonde de neuromodulation (21) ;
des moyens d'entrée pour saisir au moins un critère de filtrage ;
des moyens de filtrage configurés pour appliquer le au moins un critère de filtrage à la pluralité d'ensembles de données afin de générer un sous-ensemble filtré des ensembles de données ;
des moyens de fusion configurés pour fusionner les données spatiales des ensembles de données du sous-ensemble filtré afin de générer un ensemble de données fonctionnel ; et
des moyens de visualisation conçus pour calculer un modèle tridimensionnel (50) de l'ensemble de données fonctionnel et pour produire une image visuelle du modèle tridimensionnel (50).

2. Système médical selon la revendication 1, dans lequel les données spatiales comportent une pluralité de valeurs de données, chaque valeur de données étant affectée à un emplacement spécifique dans une grille principale (30).

3. Système médical selon la revendication 2, dans lequel les moyens de fusion sont adaptés pour calculer un score moyen de chaque valeur de données du sous-ensemble filtré qui est affecté au même emplacement dans la grille principale (30).

4. Système médical selon l'une quelconque des revendications précédentes, comportant en outre des moyens d'enregistrement pour cartographier le modèle tridimensionnel (50) de l'ensemble de données fonctionnel sur au moins une image médicale spécifique à un patient, en particulier une image par résonance magnétique, moyennant quoi les moyens de visualisation sont adaptés pour produire une image visuelle du modèle tridimensionnel (50) en association avec l'image médicale spécifique au patient (40).

5. Système médical selon l'une quelconque des revendications précédentes, dans lequel les données spatiales sont représentées par une matrice, en particulier une matrice tridimensionnelle.

6. Système médical selon l'une quelconque de revendications 2 à 5, dans lequel pour chaque point dans la grille principale, la distance jusqu'aux points voisins immédiats ne dépasse pas 1 mm, en particulier 0,75 mm, en particulier 0,5 mm, en particulier 0,25 mm, en particulier 0,1 mm.

7. Système médical selon l'une quelconque des revendications précédentes, dans lequel les moyens de filtrage sont adaptés pour recevoir au moins une expression booléenne associée aux métadonnées, moyennant quoi le sous-ensemble filtré d'ensembles de données est généré au moins en partie sur la base de l'expression booléenne.

8. Système médical selon l'une quelconque des revendications précédentes, comportant en outre des moyens de simulation pour simuler un champ de stimulation (70) de la sonde de neuromodulation (21), dans lequel les moyens de visualisation sont adaptés pour produire une image visuelle du champ de stimulation (70) par rapport au modèle tridimensionnel (50) de l'ensemble de données fonctionnel.

9. Procédé pour planifier une thérapie de neurostimulation cérébrale profonde, le procédé comportant les étapes consistant à :
mémoriser une pluralité d'ensembles de données, chaque ensemble de données comportant des métadonnées concernant des données sur le sexe, des donnés sur l'âge, des données sur des enregistrements de champs locaux, des données sur des enregistrements de microélectrodes, des données sur des structures de fibres tissulaires, des données démographiques et/ou des données anatomiques d'un patient ; et
des données spatiales concernant la répartition d'un champ de stimulation (70) d'une sonde de neuromodulation (21) ; saisir au moins un critère de filtrage ;
appliquer le au moins un critère de filtrage à la pluralité d'ensembles de données afin de générer un sous-ensemble filtré des ensembles de données ;
fusionner les données spatiales des ensembles de données du sous-ensemble filtré afin de générer un ensemble de données fonctionnel ;
calculer un modèle tridimensionnel (50) de l'ensemble de données fonctionnel ; et
produire une image visuelle du modèle tridimensionnel (50).
